(19) Europäisches Patentamt
European Patent Office
Office européen des brevets

(11) **EP 4 071 586 A1**

(12) **EUROPÄISCHE PATENTANMELDUNG**

(43) Veröffentlichungstag:
**12.10.2022 Patentblatt 2022/41**

(21) Anmeldenummer: **21167557.4**

(22) Anmeldetag: **09.04.2021**

(51) Internationale Patentklassifikation (IPC):
**G06F 3/0346** (2013.01) **G06K 9/00** (2022.01)
**G06F 3/038** (2013.01)

(52) Gemeinsame Patentklassifikation (CPC):
**G06F 3/0346; A61B 5/7475; G01S 13/82;**
**G01S 13/878; G06F 3/00; G06F 3/0383;**
A61B 5/0507; A61B 5/1172; A61B 5/318

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(71) Anmelder:
• **Fresenius Medical Care Deutschland GmbH**
**61352 Bad Homburg (DE)**
• **Fresenius Medical Care AG & Co. KGaA**
**61352 Bad Homburg (DE)**

(72) Erfinder:
• **HITZELBERGER, Christoph**
**66740 Saarlouis (DE)**
• **WITT, Waldemar**
**64289 Darmstadt (DE)**
• **POSPIECH, Martin**
**64285 Darmstadt (DE)**

(74) Vertreter: **Bardehle Pagenberg Partnerschaft mbB**
**Patentanwälte Rechtsanwälte**
**Prinzregentenplatz 7**
**81675 München (DE)**

(54) **BERÜHRUNGSLOSES BEDIENEN MEDIZINTECHNISCHER GERÄTE**

(57) Die vorliegende Erfindung betrifft Vorrichtungen und Verfahren zum berührungslosen Bedienen eines Geräts, insbesondere eines medizintechnischen Geräts. Es wird ein System bereitgestellt, welches eine mobile Zeigevorrichtung, insbesondere in Form eines Stiftes, und eine dem Gerät zugeordnete Empfangsanordnung umfasst. Die mobile Zeigevorrichtung umfasst einen Signalemitter zum Aussenden eines optischen bzw. elektromagnetischen Signals und die dem Gerät zugeordnete Empfangsanordnung umfasst Mittel zum Bestimmen eines Zeigeziels der mobilen Zeigevorrichtung relativ zu dem Gerät basierend auf dem von der mobilen Zeigevorrichtung ausgesendeten Signal, sowie Mittel zum Auslösen einer Funktion des Geräts in Abhängigkeit des Zeigeziels der mobilen Zeigevorrichtung.

Fig. 2

EP 4 071 586 A1

**Beschreibung**

**1. Technisches Gebiet**

Die vorliegende Erfindung betrifft Vorrichtungen und Verfahren zum berührungslosen Bedienen medizintechnischer Geräte.

**2. Hintergrund**

Für medizintechnische Geräte gelten besondere Hygienestandards, um die mit diesen Geräten behandelten Patienten nicht durch Übertragung von Krankheitserregern zu gefährden. Im klinischen Alltag werden solche Geräte von unterschiedlichem medizinischem Personal bedient. Dasselbe Personal ist auch mit Handlungen an verschiedenen Patienten befasst. Es besteht hierbei die Gefahr, dass Krankheitserreger wie Viren und Bakterien unabhängig von deren Quelle (Patient, Personal, Besucher, etc.) über die Bedienoberflächen der medizintechnischen Geräte, auf die sie durch Berührung aufgebracht wurden, auf Patienten und/oder medizinisches Personal übertragen werden und so die Gesundheit der Personen, insbesondere der ohnehin schon beeinträchtigten Patienten, gefährden.

Um eine (Kreuz-)Kontamination mittels solcher Geräte zu verhindern, werden üblicherweise umfangreiche Hygienemaßnahmen getroffen. Diese bestehen insbesondere im Tragen von Einmalhandschuhen für das medizinische Personal, die nach einer Handlung am Patienten und/oder einem mit diesem Patienten interagierenden medizinischem Gerät verworfen werden und durch neue sterile Handschuhe ersetzt werden. Dieser ständige Handschuhwechsel ist teuer und zeitaufwändig insbesondere in Kliniken, Intensivstationen oder Dialysezentren. Weiterhin besteht die Notwendigkeit einer hohen Handdesinfektionsrate beim medizinischen Personal. Auch diese Maßnahme ist wegen des notwendigen Desinfektionsmitteleinsatzes teuer, zeitaufwändig und schadet potenziell der Haut des Personals. Darüber hinaus besteht auch für die medizintechnischen Geräte die Notwendigkeit, sie häufig zu desinfizieren. Insbesondere Touchscreens müssen sehr häufig desinfiziert werden. Dies verursacht neben den oben erwähnten Nachteilen über die Zeit Schäden am Touchscreen durch die eingesetzten Desinfektionsmittel und der mechanischen Abnutzung durch die eigentliche Desinfektionstätigkeit aufgrund bspw. der Wischbewegung mit Tüchern beim Desinfizieren.

Es besteht also der Wunsch, Berührungen von medizinischen Geräten möglichst zu vermeiden. Im Stand der Technik sind hierzu insbesondere Verfahren der Sprachsteuerung und der Gestensteuerung bekannt. Beide Verfahren sind im klinischen Alltag jedoch wenig benutzerfreundlich und intuitiv. Sprachsteuerung ist in belebten Umgebungen mit Hintergrundgeräuschen problematisch und ist nebenbei hinsichtlich des privaten patientenbezogenen Charakters der eingegebenen Informationen nachteilig. Gestensteuerung bedarf der umfangreichen Einarbeitung des Personals und ist häufig umständlich und wenig für den dynamischen Alltag innerhalb medizinischer Einrichtungen geeignet.

Aus der US 8,570,274 B1 ist bekannt, eine Zeigestabvorrichtung zur berührungslosen Steuerung einer Benutzerschnittstelle in einem Personal-Computer zu verwenden. In der ausführbar beschriebenen Ausführungsform kommuniziert die Zeigestabvorrichtung mit am Laptop verbauten Empfängern mittels Ultraschall. Für die eingangs genannten für die Medizintechnik charakteristischen Anforderungen ist dieses Gerät jedoch nur sehr eingeschränkt von Nutzen.

Der vorliegenden Erfindung liegt deshalb die Aufgabe zugrunde, Vorrichtungen und Verfahren bereitzustellen, die eine verbesserte berührungslose Bedienung insbesondere medizintechnischer Geräte erlauben und dabei die obigen Nachteile des Standes der Technik zumindest teilweise überwinden.

**3. Zusammenfassung der Erfindung**

Diese Aufgabe wird durch die Gegenstände der unabhängigen Ansprüche gelöst. Bevorzugte Weiterbildungen sind in den abhängigen Ansprüchen angegeben.

In ihrer allgemeinsten Form betrifft die Erfindung ein System zum berührungslosen Bedienen eines Geräts. Das System umfasst eine mobile Zeigevorrichtung, insbesondere in Form eines Stiftes, und eine dem Gerät zugeordnete Empfangsanordnung.

Bei dem Gerät kann es sich insbesondere um ein medizintechnisches Gerät handeln, d.h. jede Art von Gerät, das in der Medizintechnik Einsatz findet, z.B. ein Hämodialysegerät, ein Peritonealdialysegerät, eine Infusionspumpe, eine medizinische Überwachungsvorrichtung wie bspw. ein EKG-Gerät, ein medizinisches Ultraschallgerät, eine Zellseparationsvorrichtung, eine Herz/Lungenmaschine oder jedes andere medizintechnische Gerät, das insbesondere regelmäßige Bedienereingaben im Betrieb notwendig macht.

Die mobile Zeigevorrichtung kann einen Signalemitter zum Aussenden eines Signals umfassen. Die dem Gerät zugeordnete Empfangsanordnung kann Mittel zum Bestimmen eines Zeigeziels der mobilen Zeigevorrichtung relativ zu dem medizintechnischen Gerät basierend auf dem von der mobilen Zeigevorrichtung ausgesendeten Signal, und Mittel zum Auslösen einer Funktion des Geräts in Abhängigkeit des Zeigeziels der mobilen Zeigevorrichtung umfassen.

Somit kann die Bedienung des (medizintechnischen) Geräts vollständig berührungslos erfolgen. Beispielsweise kann eine Bedienperson mit der mobilen Zeigevorrichtung auf bestimmte Teile des (medizintechnischen) Geräts deuten (z.B. einen An-/Aus-Knopf) und damit eine entsprechende Funktion am Gerät auslösen (z.B. an- bzw. ausschalten). Weitere beispielhafte Funktionen umfassen beispielsweise das Abfragen von Betriebsparameter von Komponenten des (medizintechnischen) Geräts, wie beispielsweise die Fördergeschwindigkeit einer Pumpe für eine medizinische Flüssigkeit oder das Volumen eines Flüssigkeitsbehälters, das Einstellen einer Komponente des (medizintechnischen) Geräts, wie beispielsweise die Fördergeschwindigkeit einer Pumpe für eine medizinische Flüssigkeit, die Interaktion mit einer grafischen Benutzerschnittstelle (GUI) des (medizintechnischen) Geräts, etc.. Ferner ist auch eine weitaus komplexere Funktionsbedienung denkbar, was in der detaillierten Beschreibung weiter erläutert wird.

In einer möglichen Ausgestaltung kann der Signalemitter einen optischen Signalemitter zum Aussenden eines gerichteten Lichtsignals, insbesondere eines Lasersignals, umfassen. Die Mittel zum Bestimmen des Zeigeziels der mobilen Zeigevorrichtung können einen oder mehrere optische Sensoren, insbesondere Fototransistoren und/oder Fotodioden, umfassen. Eine solche Ausführungsform nach dem Grundprinzip eines Laser-Pens lässt sich besonders einfach realisieren.

Ein Teil des Geräts kann eine Beschichtung, insbesondere eine PMMA-Beschichtung (Polymethylmetacrylat), zum Umlenken des von der mobilen Zeigevorrichtung ausgesendeten Lichtsignals zu dem einen oder den mehreren optischen Sensoren aufweisen. Dies vergrößert die Flexibilität hinsichtlich der Platzierung der Sensoren.

Das Gerät kann hierbei Bedienkomponenten und eine transparente Abdeckung für die Bedienkomponenten aufweisen, wobei die Beschichtung zumindest teilweise auf der Abdeckung angeordnet ist. Somit können auch Geräte, die sich in Gehäusen bzw. hinter Abdeckungen befinden, bedient werden, was neben optischen auch hygienetechnische Gründe hat.

In einem weiteren Aspekt der Erfindung kann der optische Signalemitter zum Aussenden von Licht im nichtsichtbaren Spektrum eingerichtet sein. Die mobile Zeigevorrichtung kann ferner einen zweiten optischen Signalemitter zum Aussenden von Licht im sichtbaren Spektrum umfassen. Hierdurch wird die Bedienung besonders robust gegenüber Umgebungslicht, wobei dennoch eine optische Rückmeldung bereitgestellt werden kann.

Als Alternative zur oben erläuterten Laserrealisierung kann der Signalemitter auch einen Signalemitter zum Aussenden eines elektromagnetischen Signals, insbesondere eines Radarsignals, umfassen. Die Mittel zum Bestimmen des Zeigeziels der mobilen Zeigevorrichtung können dann mindestens drei Empfänger zum Empfangen des von dem Signalemitter ausgesendeten elektromagnetischen Signals und Mittel zum Vergleichen des elektromagnetischen Signals mit einem, vorzugsweise in dem Gerät hinterlegten, Referenzsignal umfassen. In dieser Ausgestaltung ist die Signalkommunikation sehr robust, was zu einer besonders verlässlichen Bedienbarkeit führt.

Die dem Gerät zugeordnete Empfangsanordnung kann einen elektromagnetischen Signalemitter umfassen zum Aussenden eines Triggersignals, welches dazu geeignet ist, die mobile Zeigevorrichtung zu veranlassen, das elektromagnetische Signal von dem Signalemitter auszusenden. Für die Lagebestimmung der mobilen Zeigevorrichtung werden somit nicht, wie herkömmlich, reflektierte Radar-Signale ausgewertet, sondern direkt vom Zeigestift ausgesendete Signale. Dies erlaubt eine besonders stabile Erkennung der vom Zeigestift ausgehenden Signale. Die Mittel zum Bestimmen des Zeigeziels der mobilen Zeigevorrichtung sind hierbei vorzugsweise zum Durchführen eines FMCW-Verfahrens (frequency modulated continuous wave radar, moduliertes Dauerstrichradarverfahren) zum Bestimmen einer Distanz zwischen der mobilen Zeigevorrichtung und der dem Gerät zugeordneten Empfangsanordnung konfiguriert.

Auch kann die mobile Zeigevorrichtung ferner Mittel zur Bestimmung einer Zeigeachse der mobilen Zeigevorrichtung umfassen. Dabei kann es sich um einen zweiten Signalemitter zum Aussenden eines elektromagnetischen Signals, insbesondere eines Radarsignals, und/oder um einen Sensor zum Ermitteln einer Neigung der mobilen Zeigevorrichtung, insbesondere einen Winkelsensor und/oder einen Neigungssensor, handeln.

Unabhängig von der gewählten Art der Signalübertragung (z.B. Laser oder Radar), kann die mobile Zeigevorrichtung ferner zumindest ein Bedienelement, insbesondere zumindest einen Taster, sowie Mittel zum Modulieren einer Information auf das von dem Signalemitter ausgesendete optische oder elektromagnetische Signal in Antwort auf eine Betätigung des zumindest einen Bedienelements umfassen. Somit können prinzipiell beliebige Informationen an das Gerät übermittelt werden, z.B. Steuerbefehle.

Die mobile Zeigevorrichtung kann ferner Mittel zum Identifizieren einer Bedienperson umfassen. Diese Mittel können einen Fingerabdruckscanner oder Mittel zur Kommunikation mit einem unter einem Handschuh der Bedienperson tragbaren Fingerabdrucksensor umfassen. Somit ist eine sichere Identifikation der Bedienperson vor der Bedienung gewährleistet, was Missbrauch entgegenwirkt.

Die Mittel zum Bestimmen des Zeigeziels der mobilen Zeigevorrichtung können mit dem Gerät verbunden sein, insbesondere an dem Gerät angeordnet sein. Alternativ können die Mittel zum Bestimmen des Zeigeziels der mobilen Zeigevorrichtung entfernt von dem Gerät mit einem definierten räumlichen Bezug zu diesem angeordnet sein, beispielsweise an vordefinierten Positionen in einem Raum. Dies ermöglicht es u.a., gleich mehrere Geräte bedienbar zu machen.

Die Erfindung betrifft ferner auch die oben erläuterten Einzelkomponenten für sich genommen, nämlich eine mobile Zeigevorrichtung, ein (medizintechnisches) Gerät und/oder eine dem (medizintechnischen) Gerät zugeordnete Empfangsanordnung zur Verwendung in einem System wie oben beschrieben. Dabei weisen diese Vorrichtungen die Merkmale der jeweiligen Vorrichtung wie oben beschrieben auf.

Ebenfalls bereitgestellt wird ein Verfahren zum berührungslosen Bedienen eines Geräts, umfassend Aussenden eines Signals durch einen Signalemitter einer mobilen Zeigevorrichtung, insbesondere in Form eines Stiftes, Bestimmen eines Zeigeziels der mobilen Zeigevorrichtung relativ zu dem Gerät basierend auf dem von der mobilen Zeigevorrichtung ausgesendeten Signal durch eine dem Gerät zugeordnete Empfangsanordnung, und Auslösen einer Funktion des Geräts in Abhängigkeit des Zeigeziels der mobilen Zeigevorrichtung durch die dem Gerät zugeordnete Empfangsanordnung. Bei dem Verfahren können die mobile Zeigevorrichtung, das Gerät und/oder die dem Gerät zugeordnete Empfangsanordnung jegliche oben beschriebene Vorrichtungen sein.

Zuletzt stellt die Erfindung ein Computerprogramm bereit mit Befehlen, die bewirken, dass die mobile Zeigevorrichtung, das (medizintechnische) Gerät und/oder die dem medizintechnischen Gerät zugeordnete Empfangsanordnung die jeweiligen hier erläuterten Verfahrensschritte ausführen.

**4. Kurze Beschreibung der Zeichnungen**

Im Folgenden werden bevorzugte Ausführungsbeispiele der vorliegenden Erfindung unter Bezugnahme auf die begleitenden Figuren erläutert:

Fig. 1: Verhältnis zwischen Sende- und Empfangssignal bei der Entfernungsbestimmung mit einem FMCW-Radar;

Fig. 2: Projektion der Zeigestiftachse auf die Geräteoberfläche gemäß Ausführungsformen der Erfindung;

Fig. 3: Lagebestimmung des Zeigestifts mit durch ein Triggersignal ausgelöstem Zeigestiftsignal gemäß Ausführungsformen der Erfindung;

Fig. 4: Kodieren von Informationen im Zeigestiftsignal durch charakteristisches Unterbrechen des Signals gemäß Ausführungsformen der Erfindung;

Fig. 5: Kodieren von Informationen im Zeigestiftsignal durch Anhängen der Information an eine Modulationsperiode gemäß Ausführungsformen der Erfindung;

Fig. 6: Ermittlung der Neigung des Zeigestifts mit Winkel- bzw. Neigungssensoren gemäß Ausführungsformen der Erfindung.

**5. Detaillierte Beschreibung von bevorzugten Ausführungsformen**

Im Folgenden werden gegenwärtig bevorzugte Ausführungsbeispiele eines erfindungsgemäßen Systems zum berührungslosen Bedienen eines medizintechnischen Geräts, sowie dessen Einzelkomponenten und entsprechende Verfahren, genauer erläutert.

Allgemein gesprochen stellt die vorliegende Erfindung eine intuitive Steuerung von Geräten durch Auswerten einer Zeige- bzw. Deutbewegung auf die zu bedienende Komponente eines medizintechnischen Geräts und/oder auf eine Fläche einer grafischen Benutzerschnittstelle (GUI) desselben bereit.

Erfindungsgemäß kann hierzu eine mobile bzw. tragbare Zeigevorrichtung, insbesondere ein Zeigestift (PEN), verwendet werden. Nachfolgend werden die Prinzipien der vorliegenden Erfindung der Einfachheit halber mit Bezug auf einen Stift erläutert; es versteht sich jedoch, dass diese Prinzipien ebenso mit anderen Formfaktoren umsetzbar sind. Durch entsprechende technische Mittel wird die Lage der bevorzugten Längsachse des Stifts im Raum ermittelt. Durch rechnerische (z.B. Vektoranalyse, geometrische Berechnungen) Verlängerung dieser Achse auf die Oberfläche des zu bedienenden Geräts kann der Auftreffpunkt der Verlängerung der ermittelten Stiftachse auf der Geräteoberfläche ermittelt werden. Eine entsprechend eingerichtete Steuervorrichtung verknüpft den Auftreffpunkt mit vordefinierter Handlung, insbesondere mit dem Auslösen einer Funktion des medizintechnischen Geräts. Wird beispielsweise ermittelt, dass sich der Auftreffpunkt innerhalb der Fläche einer montierten Blutpumpe bspw. eines Dialysegeräts befindet, kann vorgesehen sein, dass auf dem GUI ein Einstellmenü für diese Pumpe (z.B. für Pumprate, Start, Stopp etc.) angezeigt wird. Auf diese Weise kann eine Bedienperson nur durch Zeigen mit einem entsprechend konfigurierten Stift auf ein Gerät Komponenten zur Bedienung auswählen. Dies umfasst auch das Auswählen von Flächen auf einem Bildschirm. Hierdurch kann beispielsweise auf Touchscreens verzichtet werden.

Zur Ermittlung der Stiftachse wird erfindungsgemäß vorgeschlagen, den Stift mit zumindest einem Signalemitter auszustatten. Nachfolgend werden bevorzugte Ausführungsbeispiele detaillierter erläutert:

Ausführungsform mit Laseremitter

In dieser Ausführungsform weist die mobile Zeigevorrichtung einen, vorzugsweise nur einen einzigen Signalemitter in Form einer gerichteten Lichtquelle auf, beispielsweise in Form eines Lasers. Analog eines Laserpens kann eine Bedienperson somit für sie sichtbar auf zu bedienende Komponenten eines Geräts zeigen, um sie anzuwählen. Hierzu weist das zu bedienende Gerät vorzugsweise optische Sensoren (z.B. Fototransistoren und/oder Fotodioden) an entsprechenden Stellen auf, die eine Bedienperson anvisieren kann.

Die Sensoren können optisch (bspw. durch Umrandungen oder Farbgebung) kenntlich gemacht sein.

In dieser Ausführungsform mit einem optischen Emitter ist es nicht notwendig, die Lage der bevorzugten Längsachse des Stifts im Raum zu ermitteln, da der Auftreffpunkt auf der Oberfläche des zu bedienenden Geräts durch entsprechende optische Sensoren am Gerät selbst unabhängig von der Lage des Stifts im Raum ermittelt wird.

Es ist denkbar, den Stift mit zwei Lichtquellen auszustatten, die in (engen) parallelen Achsen Licht abstrahlen, wovon eine im nichtsichtbaren Spektrum abstrahlt, für die die optischen Sensoren des Geräts empfindlich sind (bspw. UV Laser und sichtbares Rot). Somit kann die Bedienung robust gegenüber Umgebungslicht gestaltet werden und dennoch eine optische Rückmeldung (Zielhilfe) angeboten werden.

Auch kann eine Beschichtung angewendet werden, die optische Strahlung zu verteilten optischen Sensoren umlenkt, beispielsweise wie erläutert in: Donato Pasquariello, M. C. J. M. Vissenberg, und Galileo June Destura, "Remote-Touch: A Laser Input User-Display Interaction Technology" J. Display Technol. 4, 39-46 (2008). Eine derartige Beschichtung besteht aus einem transparenten mikrostrukturierten lichtleitenden Substrat aus Polymethylmethacrylat (PMMA). Das Substrat ist mit einer Mikrostruktur aus pyramidenförmigen Vertiefungen strukturiert. Wenn das Laserlicht auf das Substrat trifft, wird es von der Mikrostruktur im PMMA-Substrat aufgefangen und ein Teil des Laserlichts wird parallel zur Oberfläche zur Seite reflektiert und somit zu dort befindlichen optischen Sensoren umgelenkt, die somit die Position des Laserspots bestimmen können. Eine derartige Vorrichtung, die eine durchsichtige PMMA-Beschichtung umfasst, die auf der Oberfläche eines Bildschirms vorliegt, kann die Funktionalität eines Touchscreens übernehmen, ohne dass Berührungen des Bildschirms notwendig sind. Darüber hinaus sind somit günstige und skalierbare Bildschirme umsetzbar, da, insbesondere bei großen Flächen, die Anzahl der benötigten Sensoren deutlich reduziert werden kann.

Dasselbe Prinzip der optischen Umlenkung kann am ganzen Gerätekorpus angewendet werden. So ist es möglich, eine optisch transparente Abdeckung vor Bedienkomponenten eines Geräts mit o.g. PMMA-Beschichtung auszustatten, die auftretende Laserstrahlung auffängt und zu optischen Sensoren umlenkt. Somit kann die Bedienperson Komponenten, die sich hinter der Abdeckung befinden, zur Auswahl anvisieren. Medizintechnische Geräte mit optisch transparenten Abdeckungen sind beispielsweise durch das Dialysegerät 5008 der Anmelderin bekannt. Dieses ist mit zwei transparenten schwenkbaren Türen ausgestattet, die im Betrieb den sich dahinter befindlichen extrakorporalen Blutkreislauf abdecken. Dies hat neben optischen auch hygienetechnische Gründe.

Neben dem Auswählen von Komponenten ist zur Bedienung auch die Eingabe von Information zur Bedienung eines medizintechnischen Geräts wesentlich. Konventionell wird dies bspw. über Eingabeelemente wie Schalter oder Buttons eines Touchscreendisplays verwirklicht. In Ausführungsformen der Erfindung geschieht dies mit dem Zeigestift berührungslos dadurch, dass der Zeigestift zumindest über eine Eingabemöglichkeit, bspw. einen Taster, verfügt, der von der Bedienperson aktiviert werden kann. Die Aktivierung hat zur Folge, dass das ausgesendete Lichtsignal mit einer Information moduliert wird. Im einfachsten Fall kann dies ein Pulscode sein, der das Signal analog der zu übertragenden Information ein- und ausschaltet. Ebenfalls denkbar ist auch eine Amplitudenmodulation.

Die Empfänger am medizintechnischen Gerät (diese müssen nicht zwingend direkt am Gerät angeordnet sein, sondern können diesem auch räumlich entfernt zugeordnet sein; siehe weiter unten) sind zur Demodulation des Lichtsignals eingerichtet. Das medizintechnische Gerät führt entsprechend der empfangenen Information Schritte aus.

Beispielsweise kann die übertragende Information charakteristisch sein für den Zeigestift. Das medizintechnische Gerät kann dementsprechend die Bedienbarkeit erlauben, verweigern und/oder einschränken. Entsprechend kann sich auch die Bedienperson am Zeigestift identifizieren. Hier ist beispielsweise denkbar, den Zeigestift mit einem Fingerabdruckscanner als Authentifizierungsmittel zu versehen und ein mit einem eingespeicherten Fingerabdruck verknüpftes Identifikationssignal dem Lichtsignal aufzumodulieren.

Identifikationsmerkmale können, müssen aber nicht erst nach Aktivierung des Tasters mitgesendet werden. Diese können auch permanent (also periodisch ohne Aktivierungssignal) gesendet werden.

Die Authentifizierung eines Benutzers am Zeigestift kann über beliebige Mittel vollzogen werden, beispielsweise auch über Mittel für eine PIN Code Eingabe. Alternativ oder zusätzlich, im Rahmen einer Mehrfaktorauthentifizierung, kann die Authentifizierung des Benutzers mittels zusätzlicher peripherer Geräte erfolgen. Das periphere Gerät kann der Benutzer mit sich führen, es kann Teil der Zeigevorrichtung oder des medizinischen Gerätes sein oder mit der Zeigevorrichtung oder dem medizinischen Gerät im Datenaustausch stehen. Beispielsweise handelt es sich hierbei um ein Armband, eine Smartwatch, eine Smartcard oder um einen Sensor zur Erfassung biometrischer Merkmale des Benutzers.

In einer weiteren Ausführungsform ist vorgesehen, einen Fingerabdrucksensor (wie beispielsweise als Micro RF Tag von der Firma SK-Electronics Co Ltd vertrieben) zu verwenden, der unter dem Handschuh getragen werden kann und Authentifizierungsdaten per Drahtlosverbindung (z.B. NFC) an den Zeigestift überträgt, sobald die Hand der Bedienperson in die Nähe des Zeigestifts kommt. Somit wird u.a. sichergestellt, dass ein gestohlener Zeigestift unbrauchbar ist.

Der Fingerabdrucksensor beinhaltet vorzugsweise eine flächige Anordnung von o.g. passiven RFID-Tags in den Ausmaßen eines Fingerabdrucks bzw. eines eindeutigen Fingerabdruckabschnitts. Die feine Segmentierung durch die kleinen Tags ermöglicht die Erfassung der Fingerabdruck-definierenden Linien (Papillarleisten). Hierfür wird der Sensor auf der Unterseite der Fingerkuppe aufgebracht (Anpressen durch Handschuh und/oder selbstklebend). Das Sendeverhalten der einzelnen Tags wird vom jeweiligen Untergrund und dessen elektrischer Leitfähigkeit bestimmt. Durch den Kontakt bzw. den Nichtkontakt mit den Papillarleisten entsteht eine charakteristische Leitfähigkeitsverteilung, die zu entsprechendem Sendeverhalten der einzelnen Tags führt. Zur Regelung der Leitfähigkeit kann eine Paste mit hoher Benetzbarkeit zwischen Sensor und Finger vorteilhaft sein; diese kann auch adhäsiv zur Befestigung des Sensors beitragen. Ist diese Paste wärmeabhängig, insbesondere wenn sie Phasenübergänge um Temperaturen der Hautoberfläche hat, kann sichergestellt werden, dass der gescannte Finger warm, also i.d.R. der einer echten (warmen) Person ist, um Missbrauch zu erschweren. Eine Paste auf Gallium-Basis ist hinsichtlich des erreichbaren Schmelzpunkts, des elektrischen Leitverhaltens und der hohen Benetzbarkeit, die zu akkurater Abbildung des Fingerabdrucks führen, vorteilhaft. Der Zeigestift umfasst in dieser Ausführungsform Mittel zur Kommunikation mit dem Sensor (RFID-Leser). So können durch den Handschuh die Signale von den sendenden Tags ausgelesen werden. Daraus lässt sich der Fingerabdruck rekonstruieren und anschließend zur Authentifizierung nutzen.

Die Betätigung des Tasters als Eingabemittel kann einen Code übermitteln, der als Betätigung eines Auswahlmittels interpretiert werden kann. Wird beispielsweise die Blutpumpe eines Hämodialysegeräts mit dem Zeigestift ausgewählt, kann auf dem Bildschirm ein Auswahlmenu zur Einstellung dieser Pumpe angezeigt werden. Dies kann ein simples "mehr" oder "weniger" umfassen, denkbar ist aber auch das Anzeigen eines Nummernfelds mit Bestätigungsfeld. Die Bedienperson kann in schon beschriebener Weise die Auswahlfelder auf dem Display anwählen und durch Betätigen des Tasters aktivieren. So kann die Bedienperson Eingaben am medizintechnischen Gerät vornehmen und es so vollständig ohne Berührung bedienen.

Ausführungsform mit zwei Radaremittern

Neben optischen Signalen können auch andere elektromagnetische Signale verwendet werden. Besonders vorteilhaft sind hierbei elektromagnetische Signale mit kleinen Wellenlängen wie Radar, mit denen kleinste Bewegungsamplituden detektierbar sind. Radarsensorik besteht im Wesentlichen aus Signalerzeugern und Empfängern jeweils in Form von Antennen oder Empfängerspulen.

Im Folgenden wird eine Ausführungsform der Erfindung anhand des Beispiels Radar für elektromagnetische Wellen erklärt. Es ist für den Fachmann aber ersichtlich, dass auch andere elektromagnetische Wellen genutzt werden können, bzw. dass der Begriff Radar weit gefasst werden kann im Hinblick auf den Wellenlängenbereich.

Im Stand der Technik wird Radarsensorik regelmäßig zur Abstands- und Bewegungserkennung genutzt, in dem ein ausgesendetes Signal in Bezug auf ein von einem Objekt reflektiertes Signal hinsichtlich Signalamplitude und Phasenbeziehung (zeitlicher Versatz) ausgewertet wird. Eine bekannte Anwendung ist die Geschwindigkeitsüberwachung von Fahrzeugen. In letzter Zeit wird aber auch Radarsensorik kommerziell angeboten, die Auswertungen von kleinsten Bewegungsamplituden zulässt und somit auch zur Messung von Vitalparametern von Patienten angewendet werden kann (siehe bspw. WO 18/167777A1). Die dazugehörigen Sende- und Empfangseinheiten sind ausreichend kompakt, um auch in einer Vielzahl in ein medizintechnisches Gerät eingebaut werden zu können. Ein Anwendungsfeld von Radarsensorik ist die hochgenaue Abstandsmessung.

Zur Bestimmung der Distanz zu einem Objekt kann beispielsweise das Prinzip des frequenzmodulierten Dauerstrichradar (FMCW Radar) eingesetzt werden (siehe grundlegende Erläuterungen bspw. in https://www.radartutorial.eu/02.basics/Frequenzmodulierte%20Dauerstrichradargerä te.de.html und https://www.radartutorial.eu/02. basics/pubs/FMCW-Radar.de.pdf). Grundlegend für das Prinzip dieser Distanzmessung ist der Vergleich eines reflektierten Radarsignals mit einer Referenz, nämlich i.d.R. eines ausgesendeten Radarsignals. Über die Ausbreitungsgeschwindigkeit von Radarwellen (= $c_0$ Lichtgeschwindigkeit) und die Laufzeit zwischen Aussendung und Empfang der Reflektion kann die Distanz bestimmt werden. Aufgrund der sehr hohen Ausbreitungsgeschwindigkeit und der in industriellen Anwendungen vergleichsweise nahen Entfernung ist die direkte Bestimmung der Laufzeit technisch schwierig. Ein Ausweg bietet das o.g. FMCW Verfahren, das als Anregungssignal ein kontinuierlich in der Frequenz veränderliches Signal nutzt und statt der Laufzeit die Frequenzdifferenz zwischen anregendem und reflektiertem Signal zur Distanzmessung verwendet, was technisch präziser und weniger aufwendig machbar ist.

In dem in der oben genannten Veröffentlichung gezeigten Beispiel wird die Distanz zwischen Sender/Empfänger und Messobjekt nach dem folgenden Zusammenhang bestimmt:

$$R = \frac{c_0\,|\Delta t|}{2} = \frac{c_0\,|\Delta f|}{2\left(\frac{d(f)}{d(t)}\right)}$$

wobei $c_0$ = Lichtgeschwindigkeit = $3 \times 10^8$ $m/s$; $\Delta t$ = Laufzeit [s]; $\Delta f$ = gemessene Frequenzdifferenz [Hz] ; $R$ = Entfernung Antenne - Objekt [m]; $d(f)/d(t)$ = Frequenzhub pro Zeiteinheit

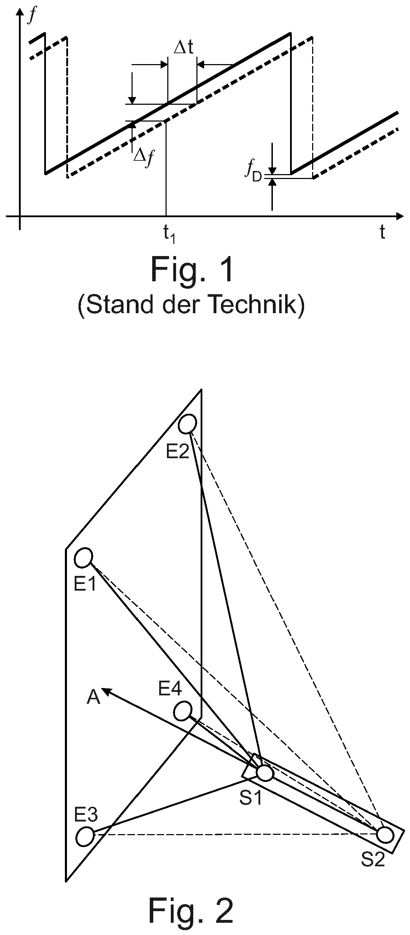

Hierbei ist der Frequenzhub pro Zeiteinheit die Steigung der Frequenzmodulation, wie in Fig. 1 dargestellt. Bei dem durchgezogen dargestellten Signal handelt es sich um ein Sende-/Referenzsignal und bei dem gestrichelt dargestellten um ein reflektiertes Signal.

Hinsichtlich des Prinzips der radargestützten Distanzmessung gibt es bereits Lösungen aus dem Stand der Technik. Beispielhaft seien DE102019002278A1, WO2016/204641A1, WO20127177A1, WO9908128A1 und WO07009833 A1 genannt.

Hierbei stellt die FMCW Messung nur eine beispielhafte Ausführungsform dar.

Um das oben beschriebene Prinzip der berührungslosen Steuerung auch mit Radarsensorik umsetzbar zu machen, verfügt das medizinische Gerät in einer Ausführungsform über mindestens drei (zur Bestimmung der Senderpositionen im Raum) für Radarsignale (bzw. elektromagnetische Signale bestimmter Wellenlängen) empfindliche Empfänger und/oder mindestens einen Sender für ein elektromagnetisches Signal. Weiterhin verfügt der Zeigestift über mindestens einen Sender, sowie Mittel zur Bestimmung der Lage der Zeigeachse in Bezug auf den Sender. In einer Ausführungsform ist dieses Mittel wenigstens ein weiterer räumlich vom ersten Sender entfernter zweiter Sender. In einer weiteren Ausführungsform umfasst der Zeigestift auch einen Empfänger. In allen Ausführungsformen umfasst der Zeigestift ein Steuergerät, sowie eine Energiequelle. In weiteren Ausführungsformen umfasst der Sender auch Eingabemittel zur Eingabe von Information, analog zur weiter oben beschriebenen Ausführungsformen mit Laser-Emitter.

Vorzugsweise beruhen die auf der Auswertung elektromagnetischer Strahlen basierenden Ausführungsformen der Erfindung auf der Bestimmung der Lage der Achse des Zeigestifts in Bezug auf das zu bedienende Gerät und daraus abgeleitet der Bestimmung der Position der vom Bediener anvisierten Komponente des medizintechnischen Geräts, das sich durch die Projektion der so bestimmten Achse auf die Geräteoberfläche bestimmen lässt. Dies ist in Fig. 2 verdeutlicht.

In diesem Beispiel weist das medizintechnische Gerät vier Empfänger (E1-E4) auf. Ein Sender am medizintechnischen Gerät ist hier nicht gezeigt. Der Zeigestift weist zwei Sender S1 und S2 auf, die auf der Längsachse des Stabs angeordnet sind. Über das weiter unten beschriebene Verfahren lassen sich die Entfernung der Sender S1 und S2 zu jeweils allen Empfängern bestimmen (angedeutet durch die durchgezogenen und unterbrochenen Linien von den Empfängern zu den Sendern) und somit durch rein geometrische Berechnungen im medizintechnischen Gerät die Lage des Zeigestifts zum medizintechnischen Gerät. Somit lässt sich auch die Projektion der Längsachse auf die Geräteoberfläche (Pfeil zu A) bestimmen. Die Position ergibt sich im Beispiel nach Fig. 2 daraus, dass die Entfernungen der Sender S1 und S2 zu den Empfängern E1-E4 bestimmt werden können und sich somit geometrisch eindeutige Pyramiden ergeben, deren Grundflächen von dem Rechteck gebildet wird, an dessen Ecken sich die Empfänger E1-E4 befinden und deren jeweilige Spitze durch die Sender S1 und S2 gebildet werden. Die Lage der Spitzen ist durch die Entfernungen zu den einzelnen Empfängern definiert. Die Länge der Pyramidenkanten entspricht gerade der jeweiligen bestimmten Entfernung. Somit können beispielsweise die Koordinaten der Sender S1 und S2 in einem Bezugsraum bestimmt werden. Mit diesen Koordinaten kann ein Vektor bestimmt werden, auf dem die Sender S1 und S2 liegen. Durch die mathematische Bestimmung des Schnittpunkts dieses Verbindungsvektors mit der Ebene im Bezugsraum, auf der die Empfänger E1-E4 liegen, kann somit der Auftreffpunkt A ermittelt werden. In Fig. 2 werden vier Empfänger gezeigt. Zur Bestimmung des Auftreffpunkts sind wenigstens drei Empfänger notwendig.

In Ausführungsformen der vorliegenden Erfindung werden, um stabile vom Zeigestift ausgehende Signale zu erhalten, keine reflektierten Radar-Signale ausgewertet, sondern direkt von einem Zeigestift ausgesendete Signale, um durch entsprechende Signalauswertung die Lage der Achse des Zeigestifts zu bestimmen. Zur Bestimmung der Distanz zum Sendepunkt ist es wesentlich, dass sich das vom Zeigestift ausgesendete Signal wie ein reflektiertes Signal verhält. Um dies zu erreichen, ist es vorgesehen, dass vom medizintechnischen Gerät Triggersignale ausgesendet werden, die empfangen durch den Zeigestift die Aussendung der Radarsignale von den beiden Sendern initiieren.

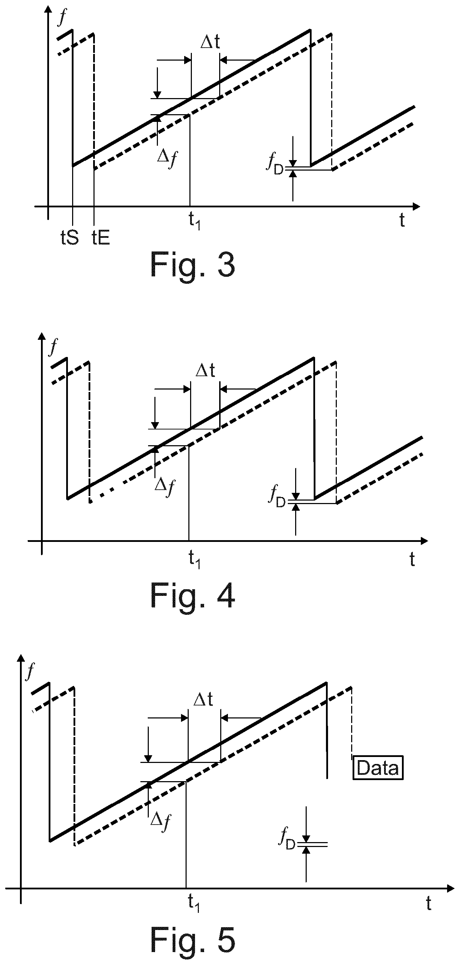

Bezugnehmend auf die FMCW-Ausführungsform und Fig. 3 umfasst dieses Verfahren folgende Schritte:

Zum Zeitpunkt tS sendet die Sendevorrichtung ein (beliebiges) elektromagnetisches Triggersignal. Der Zeitpunkt tS wird mit dem durchgezogen dargestellten Signal verknüpft. Das durchgezogen dargestellte Signal liegt nur als Referenz-Signalverlauf im medizintechnischen Gerät vor und wird nicht gesendet. Mit diesem gespeicherten Referenzsignal wird das gestrichelt dargestellte Signal verglichen, bspw. die Differenzfrequenz bestimmt, woraus sich die Entfernung zwischen Sender und Empfänger ableiten lässt. Das Triggersignal kann periodisch ausgesendet werden, um eine permanente Positionsbestimmung zu ermöglichen.

Zum Zeitpunkt (tS+tE)/2 empfängt die Empfangsvorrichtung des Zeigestifts das Triggersignal und initiiert umgehend die Aussendung des charakteristischen gestrichelt dargestellten Signals, welches zum Zeitpunkt tE an einem der Empfänger des medizintechnischen Geräts detektiert wird. Durch den oben beschriebenen Vergleich kann entsprechend die Entfernung zwischen Sender und dem jeweiligen Empfänger bestimmt werden.

Denkbar ist auch, dass der Triggervorgang vom Zeigestift angestoßen wird, beispielsweise dadurch, dass eine Bewegungserkennung im Zeigestift eine Bewegung erkennt, über einen (beliebigen) Sender ein Aktivierungssignal aussendet, welches vom medizintechnischen Gerät empfangen wird, woraufhin dieses das Triggersignal in oben beschriebener Weise aussendet.

Auf diese Weise findet kein permanentes Triggering statt, sondern nur bei erkannter Bewegung des Zeigestifts.

Es ist hierbei nicht wesentlich, dass Sender und Empfänger am medizintechnischen Gerät an derselben Stelle angeordnet sind. Demzufolge kann ein einziger Sender am medizintechnischen Gerät verwendet werden, der vorteilhaft dieselbe Entfernung zu allen Empfängern am medizintechnischen Gerät aufweist. Es ist allerdings genauso möglich, dass kombinierte Sende- und Empfangsmittel (SE1-SE3, SE4) verwendet werden, die nacheinander das beschriebene Verfahren ausführen, um jeweils die Entfernung zum jeweiligen Sender des Zeigestifts zu bestimmen.

Wenn der Zeigestift zwei Sender S1 und S2 aufweist, können verfahrenstechnisch die Entfernungen zu Sender 1 und Sender 2 nacheinander bestimmt werden, oder bei Verwendung nicht überlappender Frequenzbereiche auch gleichzeitig. Die Dauer einer Messung, also ca. die Modulationsperiode T, ist hierbei so kurz bemessen, dass zwischenzeitliche Bewegungen des Zeigestifts keine Rolle spielen. Zwar ist typisch für die FMCW-Messung, dass die Modulationsperiode T viel größer ist als die Laufzeit des Signals, aufgrund der Ausbreitungsgeschwindigkeit (Lichtgeschwindigkeit), der kurzen Entfernung (wenige Meter maximal) und des Frequenzbereichs (mehrere GHz; bspw. 85-100GHz) ergeben sich hierfür allerdings so kleine Werte, dass die Bestimmung der Entfernung innerhalb kürzester Zeitperioden (bspw. < 1 Millisekunde) möglich ist.

Zur Übertragung von zusätzlichen Informationen zwischen dem Zeigestift und dem medizintechnischen Gerät kann ebenfalls das gesendete Signal verwendet werden. Dies kann beliebig moduliert werden bzw. modifiziert oder ergänzt werden. So kann vorgesehen sein, dass das Signal des Zeigestifts charakteristisch unterbrochen ist und so den Zeigestift identifiziert. Zur Bestimmung der Distanz ist es nicht notwendig, dass das gesendete Signal vollständig alle Frequenzen enthält, wie in Fig. 4 verdeutlicht.

In diesem Beispiel umfasst das gestrichelt dargestellte Signal des Zeigestifts zu Beginn einige Lücken, während derer das Signal unterbrochen ist, die Bestimmung des Zeitversatzes über die Frequenzdifferenz der beiden Signalverläufe erfolgt aber erst zu einem späteren Zeitpunkt. Die Lücken im Rücksendesignal können Informationen, bspw. über den Benutzer oder den Zeigestift kodieren. Ebenso ist es möglich, Information einer Modulationsperiode anzuhängen, wie dies in Fig. 5 dargestellt ist.

Die Daten können in beliebiger Weise auf das Trägersignal aufmoduliert werden, beispielsweise als Frequenz- oder Amplituden oder in einem FSK Verfahren. Auch ist die Basisfrequenz beliebig. Im obigen Beispiel liegt diese innerhalb des Spektrums der Radarfrequenzen, was zur Folge hat, dass für die Distanzbestimmung und die Datenübertragung dieselbe Sendevorrichtung verwendet werden kann. Es ist auch vorstellbar, dass der Zeigestift über einen zusätzlichen Sender, der nur zur Datenübertragung eingerichtet ist, verfügt. Dieser kann in der Sendefrequenz so gewählt werden, dass er sich nicht mit den ausgestrahlten Radarwellen überschneidet. Somit kann die Datenübertragung auch zeitgleich zur Distanzmessung stattfinden.

Wie schon in der Ausführungsform mit Lasersender beschrieben, kann in analoger Weise der Zeigestift mit einem Taster und Authentifizierungsmitteln ausgestattet sein. Die Datenübertragung geschieht hierbei wie oben beschrieben über die Sende/Empfängermittel des Zeigestifts und des medizintechnischen Geräts, die für elektromagnetische Strahlung empfindlich sind und keine optischen Sensoren sind.

## Ausführungsform mit einem Radaremitter und Winkelsensoren

In einer weiteren Ausführungsform umfasst der Zeigestift einen einzigen Sender und zusätzliche Sensoren zur Bestimmung der Neigung des Zeigestifts im Raum und des Winkels, den die Achse des Zeigestifts mit dem Erdmagnetfeld annimmt. Für beide Größen kennt der Fachmann hochpräzise Sensoren, die auch in genügend kleinen Ausführungen erhältlich sind, um in einem Zeigestift nach der vorliegenden Erfindung integriert werden zu können. Beispielhaft sollen hier der integrierte 360° Winkelsensor TLE5012B der Firma Infineon, sowie der Neigungssensor STMicroelectronics IIS2ICLX genannt werden.

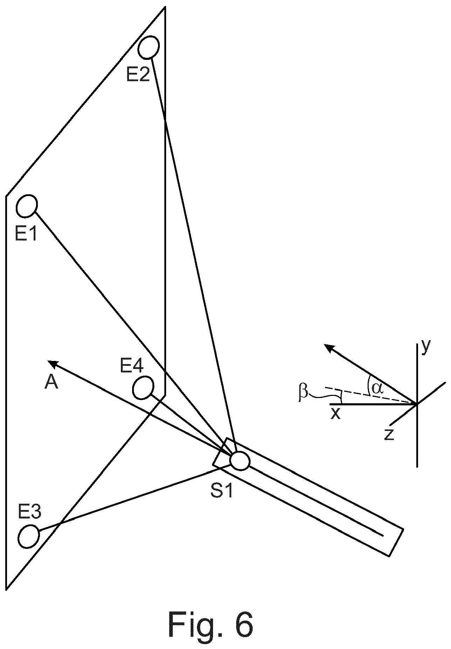

Sind dem medizintechnischen Gerät die Lage und Position in Bezug auf die Raumachsen ebenfalls bekannt (was unter Zuhilfenahme äquivalenter Sensoren bewerkstelligt werden kann), lassen sich durch Übertragung dieser Informationen der Auftreffpunkt der Projektion der Zeigestiftachse ebenfalls eindeutig bestimmen, wie in Fig. 6 verdeutlicht ist.

Durch die im Zeigestift integrierten Winkel- und/oder Neigungssensoren lassen sich die Winkel $\alpha$ und $\beta$ in Bezug auf die Referenzachsen x, y und z eindeutig bestimmen. Wird diese Information an das medizinische Gerät übertragen und die relative Position der Geräteoberfläche zu denselben Referenzachsen ist bekannt, kann ein entsprechend dazu programmiertes Steuergerät den Auftreffpunkt unter Berücksichtigung der ebenfalls bestimmten Position des Senders S1 beispielsweise analog des oben beschrieben Verfahrens berechnen.

Da sich die relative Position der Geräteoberflächen zu o.g. Referenzachsen durch praxisübliche Translationen oder Rotationen von Gerät, bzw. einzelnen Geräteteilen, insbesondere durch eine Neigung des Bildschirms, ändern kann, ist eine dynamische Berechnung des Auftreffpunkts zumindest vorteilhaft. Diese kann erreicht werden, indem die Gerätegeometrie, insbesondere in Relation zum Empfänger, modelliert und mit der Berechnung der Strahlengeometrie verknüpft wird. Die modellierte Gerätegeometrie erlaubt zusätzlich die Definition von aktiven Interaktionspunkten/- flächen, die Geräteelementen entsprechen, die ausgewählt und beeinflusst werden können. Abhängig von Nutzer und aktuellen Gerätebetriebsstatus können die aktiven Elemente verändert werden, wodurch eine kontext-adaptive Steuerung ermöglicht wird.

Ausführungsform mit Raum-/Gebietsüberwachung

In den oben beschriebenen Ausführungsformen sind die Empfängervorrichtungen unmittelbar am medizintechnischen Gerät angeordnet. Dies ist jedoch nicht zwingend notwendig; wesentlich ist nur, dass die Empfängervorrichtungen dem medizintechnischen Gerät zugeordnet sind bzw. die empfangenen Signale einem medizintechnischen Gerät zugeordnet werden können. Beispielsweise ist es denkbar, die Empfängervorrichtungen nicht als integraler Bestandteil eines zu bedienenden Geräts anzubringen, sondern innerhalb eines Raums oder eines Gebiets, im dem sich mehrere Geräte befinden können.

Beispielsweise können Empfänger in den Raumecken angebracht werden, die in der o.g. Weise die Position und die Zeigeachse des Zeigestifts bestimmen. Sind Raumgeometrie, Ausmaße, Position und Ausrichtungen der Geräte bekannt, kann so bestimmt werden, welches Gerät und welche Komponente anvisiert wird und über eine zentrale Steuerung Steuerbefehle an diese ausgegeben werden. Hierzu sind diese datentechnisch vernetzt. Auch hier kann über die o.g. Weise der Zeigestift bestimmten Personen und/oder zu bedienenden Geräten zugeordnet werden, um die Bedienung zu ermöglichen oder einzuschränken.

Diese Ausführungsform ist insbesondere für große, ortsfest installierte Industrieanlagen vorteilhaft, bei denen sich die Position und Ausrichtung der zu bedienenden Geräte oder Komponenten nicht ändern.

Vorstellbar ist, dass dieses Prinzip nicht nur zur Bedienung, sondern auch zur Informationsgewinnung verwendet wird. Hierbei werden Informationen über anvisierte Anlagenteile einem mitgeführten Gerät (Tablet, Smartphone) übermittelt. Diese können beispielsweise Betriebsdaten, Wartungsdaten oder generelle Informationen sein. Ein solches Verfahren ist insbesondere bei großen Industrieanlagen vorteilhaft, bei denen Informationen über Anlagenkomponenten zentral angezeigt werden, im Einsatz aber Informationen vor Ort abgefragt werden sollen.

Es ist denkbar, die Zeigevorrichtung in der mitgeführten Anzeigevorrichtung zu integrieren. So könnte bspw. mit dem Smartphone auf eine Anlagenkomponente gedeutet werden. Mit Hilfe entsprechender Sensoren kann dies in der o.g. Weise detektiert werden, und über eine vernetzte Zentralsteuerung können in der Folge Informationen über die entsprechende Komponente an das Smartphone gesendet werden.

Für die grundlegende Idee der vorliegenden Erfindung ist es unwesentlich, mit welchem Verfahren die Distanz zwischen Sender und Empfänger bestimmt wird.

Denkbar ist auch eine Amplitudenauswertung von gesendeten elektromagnetischen Wellen der Sender S1 und S2 und Triangulation.

Es ist grundsätzlich vorstellbar, gerichtete Sendeantennen für die Sender S1 und S2 zu verwenden, dies ist aber keine Voraussetzung.

Zur Vermeidung von ungewollten Interferenzen mehrerer Zeigestäbe innerhalb eines Raums können die Frequenzbereiche der Sender S1 und S2 für jeden Zeigestift so gewählt werden, dass sie sich nicht mit denen andere Zeigestäbe überschneiden. Ein empfangendes medizintechnisches Gerät erkennt den empfangenen Frequenzbereich und wählt entsprechend ein korrespondierendes Referenzsignal. Es kann dazu vorgesehen sein, dass im medizintechnischen Gerät das empfangene Spektrum durch Signalfilter in entsprechende Frequenzbereiche aufgeteilt wird. So kann beispielsweise auch eine simultane Bedienung eines Geräts mit mehreren Zeigestäben realisiert werden.

Eine andere Möglichkeit, ungewollte Interferenz zu vermeiden, besteht im Zeitmultiplexen des Verfahrens für mehrere Zeigestifte. Dies sieht vor, jedem Zeigestift einen periodischen Zeitbereich zuzuweisen, in dem er Signale aussenden kann. Dies kann durch eine zentrales Zeitsignal realisiert werden, das ausgesendet und von jedem Zeigestift empfangen wird. Die Zeigestifte können dann derart konfiguriert sein, nur zu bestimmten Zeitslots aktiv zu sein, zu denen kein anderer Zeigestift aktiv ist.

Durch die beschriebenen Methoden bemerkt jedes Gerät, ob ein Zeigestift eines seiner Komponenten anvisiert oder nicht, was bedeuten kann, dass er ein anderes Gerät anvisiert. Dementsprechend wird es nur im ersten Fall "aktiv". So können mit einem einzigen Zeigestift eine Vielzahl der unterschiedlichsten Geräte angesteuert werden.

**Patentansprüche**

1. Ein System zum berührungslosen Bedienen eines Geräts, umfassend:

   eine mobile Zeigevorrichtung, insbesondere in Form eines Stiftes, umfassend:
   einen Signalemitter zum Aussenden eines Signals; und
   eine dem Gerät zugeordnete Empfangsanordnung, umfassend:

      Mittel zum Bestimmen eines Zeigeziels der mobilen Zeigevorrichtung relativ zu dem Gerät basierend auf dem von der mobilen Zeigevorrichtung ausgesendeten Signal; und
      Mittel zum Auslösen einer Funktion des Geräts in Abhängigkeit des Zeigeziels der mobilen Zeigevorrichtung.

**2.** Das System nach Anspruch 1, wobei das Gerät ein medizintechnisches Gerät, insbesondere ein Hämodialysegerät, ein Peritonealdialysegerät, eine Infusionspumpe, eine medizinische Überwachungsvorrichtung, ein EKG-Gerät, ein medizinisches Ultraschallgerät, eine Zellseparationsvorrichtung oder eine Herz/Lungenmaschine umfasst.

**3.** Das System nach Anspruch 1 oder 2, wobei der Signalemitter einen optischen Signalemitter zum Aussenden eines gerichteten Lichtsignals, insbesondere eines Lasersignals, umfasst; und wobei die Mittel zum Bestimmen des Zeigeziels der mobilen Zeigevorrichtung einen oder mehrere optische Sensoren, insbesondere Fototransistoren und/oder Fotodioden, umfassen.

**4.** Das System nach Anspruch 3, wobei zumindest ein Teil des Geräts eine Beschichtung, insbesondere eine PMMA-Beschichtung, zum Umlenken des von der mobilen Zeigevorrichtung ausgesendeten Lichtsignals zu dem einen oder den mehreren optischen Sensoren aufweist; wobei das Gerät vorzugsweise Bedienkomponenten und eine transparente Abdeckung für die Bedienkomponenten aufweist, und wobei die Beschichtung zumindest teilweise auf der Abdeckung angeordnet ist.

**5.** Das System nach irgendeinem der Ansprüche 2-4, wobei der optische Signalemitter zum Aussenden von Licht im nichtsichtbaren Spektrum eingerichtet ist und wobei die mobile Zeigevorrichtung ferner einen zweiten optischen Signalemitter zum Aussenden von Licht im sichtbaren Spektrum umfasst.

**6.** Das System nach Anspruch 1 oder 2, wobei der Signalemitter wenigstens einen Signalemitter (S1) zum Aussenden eines elektromagnetischen Signals, insbesondere eines Radarsignals, umfasst; und wobei die Mittel zum Bestimmen des Zeigeziels der mobilen Zeigevorrichtung umfassen:

mindestens drei Empfänger (E1-E4) zum Empfangen des von dem wenigstens einen Signalemitter (S1) ausgesendeten elektromagnetischen Signals; und Mittel zum Vergleichen des elektromagnetischen Signals mit einem vorzugsweise in dem Gerät hinterlegten Referenzsignal.

**7.** Das System nach Anspruch 6, wobei die dem Gerät zugeordnete Empfangsanordnung ferner einen elektromagnetischen Signalemitter umfasst zum Aussenden eines Triggersignals, welches dazu geeignet ist, die mobile Zeigevorrichtung zu veranlassen, das elektromagnetische Signal von dem wenigstens einem Signalemitter (S1) auszusenden.

**8.** Das System nach Anspruch 6 oder 7, wobei die Mittel zum Bestimmen des Zeigeziels der mobilen Zeigevorrichtung zum Durchführen eines FMCW-Verfahrens zum Bestimmen einer Distanz zwischen dem wenigstens einem Signalemitter der mobilen Zeigevorrichtung und der dem Gerät zugeordneten Empfangsanordnung konfiguriert sind.

**9.** Das System nach irgendeinem der Ansprüche 6-8, wobei die mobile Zeigevorrichtung ferner Mittel zur Bestimmung einer Zeigeachse der mobilen Zeigevorrichtung umfasst; wobei die Mittel zur Bestimmung der Zeigeachse vorzugsweise wenigstens einen zweiten Signalemitter (S2) zum Aussenden eines elektromagnetischen Signals, insbesondere eines Radarsignals, umfassen; und/oder wobei die Mittel zur Bestimmung der Zeigeachse vorzugsweise einen Sensor zum Ermitteln einer Neigung der mobilen Zeigevorrichtung, insbesondere einen Winkelsensor und/oder einen Neigungssensor, umfassen.

**10.** Das System nach irgendeinem der vorhergehenden Ansprüche, wobei die mobile Zeigevorrichtung ferner umfasst:

zumindest ein Bedienelement, insbesondere zumindest einen Taster; und Mittel zum Modulieren einer Information auf das von dem Signalemitter ausgesendete Signal in Antwort auf eine Betätigung des zumindest einen Bedienelements.

**11.** Das System nach irgendeinem der vorhergehenden Ansprüche, wobei die mobile Zeigevorrichtung ferner Mittel zum Identifizieren einer Bedienperson umfasst, wobei die Mittel zum Identifizieren vorzugsweise einen Fingerabdruckscanner umfassen; und/oder wobei die Mittel zum Identifizieren vorzugsweise Mittel zur Kommunikation mit einem unter einem Handschuh der Bedienperson tragbaren Fingerabdrucksensor umfassen.

**12.** Das System nach irgendeinem der vorhergehenden Ansprüche, wobei die Mittel zum Bestimmen des Zeigeziels der mobilen Zeigevorrichtung mit dem Gerät verbunden, insbesondere an dem Gerät angeordnet, sind; oder wobei die Mittel zum Bestimmen des Zeigeziels der mobilen Zeigevorrichtung entfernt von dem Gerät mit einem definierten räumlichen Bezug zu diesem angeordnet sind.

**13.** Eine mobile Zeigevorrichtung, ein Gerät oder eine dem Gerät zugeordnete Empfangsanordnung, welche bzw. welches die Merkmale der jeweiligen Vorrichtung in irgendeinem der Ansprüche 1-12 aufweist.

14. Ein Verfahren zum berührungslosen Bedienen eines Geräts, umfassend:

> Aussenden eines Signals durch einen Signalemitter einer mobilen Zeigevorrichtung, insbesondere in Form eines Stiftes;
> Bestimmen eines Zeigeziels der mobilen Zeigevorrichtung relativ zu dem Gerät basierend auf dem von der mobilen Zeigevorrichtung ausgesendeten Signal durch eine dem Gerät zugeordnete Empfangsanordnung; und
> Auslösen einer Funktion des Geräts in Abhängigkeit des Zeigeziels der mobilen Zeigevorrichtung durch die dem Gerät zugeordnete Empfangsanordnung;
> wobei die mobile Zeigevorrichtung, das Gerät und/oder die dem Gerät zugeordnete Empfangsanordnung vorzugsweise Vorrichtungen nach Anspruch 13 sind.

15. Ein Computerprogramm, umfassend Befehle, die bewirken, dass die mobile Zeigevorrichtung, das Gerät und/oder die dem Gerät zugeordnete Empfangsanordnung nach Anspruch 13 die jeweiligen Verfahrensschritte nach Anspruch 14 ausführt.

Fig. 1
(Stand der Technik)

Fig. 2

Fig. 3

Fig. 4

Fig. 5

Fig. 6

Europäisches
Patentamt
European
Patent Office
Office européen
des brevets

## EUROPÄISCHER RECHERCHENBERICHT

**Nummer der Anmeldung**

EP 21 16 7557

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2012/326979 A1 (GEISERT FRANZISKA [DE] ET AL) 27. Dezember 2012 (2012-12-27) | 1,3,10, 12-15 | INV. G06F3/0346 |
| Y | * Absätze [0036] – [0038]; Ansprüche 6-13; Abbildung 1 * | 2 | G06K9/00 G06F3/038 |
| | ----- | | |
| X | WO 2008/042220 A2 (NELLCOR PURITAN BENNETT LLC [US]; MUSIC DOUG [US] ET AL.) 10. April 2008 (2008-04-10) | 1-3,10, 12-15 | |
| Y | * Seiten 14-18; Abbildungen 4,5 * | 2 | |
| | ----- | | |

**RECHERCHIERTE SACHGEBIETE (IPC)**

G06F
G06K

Der vorliegende Recherchenbericht wurde für alle Patentansprüche erstellt

| Recherchenort | Abschlußdatum der Recherche | Prüfer |
|---|---|---|
| **München** | **29. September 2021** | **Kahn, Svenja** |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

Europäisches
Patentamt

European
Patent Office

Office européen
des brevets

Nummer der Anmeldung

EP 21 16 7557

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

**Siehe Ergänzungsblatt B**

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**1-3, 10, 12-15**

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT**
**DER ERFINDUNG**
**ERGÄNZUNGSBLATT B**

**Nummer der Anmeldung**

**EP 21 16 7557**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

```
1. Ansprüche: 1-3, 10, 12-15


    (i) Der Gegenstand des Anspruchs 1 (bzw. der Ansprüche
    13-15)
    (ii) Darüber hinaus der Gegenstand der Ansprüche 3, 10 und
    12
    (iii) Darüber hinaus der Gegenstand des Anspruchs 2
                            ---


2. Anspruch: 4


    (i) Der Gegenstand des Anspruchs 1
    (iv) Darüber hinaus der Gegenstand des Anspruchs 4
                            ---


3. Anspruch: 5


    (i) Der Gegenstand des Anspruchs 1
    (v) Darüber hinaus der Gegenstand des Anspruchs 5
                            ---


4. Ansprüche: 6-9


    (i) Der Gegenstand des Anspruchs 1
    (vi) Darüber hinaus der Gegenstand der Ansprüche 6-9
                            ---


5. Anspruch: 11


    (i) Der Gegenstand des Anspruchs 1
    (vii) Darüber hinaus der Gegenstand des Anspruchs 11
                            ---
```

## ANHANG ZUM EUROPÄISCHEN RECHERCHENBERICHT
## ÜBER DIE EUROPÄISCHE PATENTANMELDUNG NR.

EP 21 16 7557

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-09-2021

| Im Recherchenbericht angeführtes Patentdokument | Datum der Veröffentlichung | Mitglied(er) der Patentfamilie | Datum der Veröffentlichung |
|---|---|---|---|
| US 2012326979 A1 | 27-12-2012 | DE 102012208748 A1<br>US 2012326979 A1 | 27-12-2012<br>27-12-2012 |
| WO 2008042220 A2 | 10-04-2008 | WO 2008042219 A2<br>WO 2008042220 A2 | 10-04-2008<br>10-04-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8570274 B1
- WO 18167777 A1
- DE 102019002278 A1
- WO 2016204641 A1
- WO 20127177 A1
- WO 9908128 A1
- WO 07009833 A1

**In der Beschreibung aufgeführte Nicht-Patentliteratur**

- **DONATO PASQUARIELLO ; M. C. J. M. VISSENBERG ; GALILEO JUNE DESTURA.** Remote-Touch: A Laser Input User-Display Interaction Technology. *J. Display Technol.,* 2008, vol. 4, 39-46